# EUROPEAN PATENT SPECIFICATION

(11) **EP 4 258 920 B1**
(45) Date of publication and mention of the grant of the patent: **11.12.2024**
(21) Application number: 21839110.0
(22) Date of filing: 14.12.2021
(51) Int. Cl.: A24F 40/53, A24F 40/65, G08B 25/00, G08B 13/14, A24F 47/00, A61M 15/06, G08B 25/10

(54) **AEROSOL GENERATION DEVICE COMPRISING A GEOLOCATION CHIP AND ASSOCIATED AEROSOL GENERATION ASSEMBLY**
AEROSOLERZEUGUNGSVORRICHTUNG MIT EINEM GEOLOKATIONSCHIP UND ZUGEHÖRIGE AEROSOLERZEUGUNGSANORDNUNG
DISPOSITIF DE GÉNÉRATION D'AÉROSOL COMPRENANT UNE PUCE DE GÉOLOCALISATION ET ENSEMBLE DE GÉNÉRATION D'AÉROSOL

(30) Priority: 14.12.2020 EP 20213885
(43) Date of publication of application: 18.10.2023
(73) Proprietor: JT International S.A., 1202 Genève (CH)
(72) Inventor: YAMAGUCHI, Akira, 1207 GENEVE (CH)
(74) Representative: Lavoix
(86) International application number: PCT/EP2021/085607
(87) International publication number: WO 2022/128990

(56) References cited:
- EP-A1- 3 342 442
- US-A1- 2015 181 945
- US-A1- 2019 387 796

## Description

### FIELD OF THE INVENTION

The present invention concerns an aerosol generation device.

The present invention also concerns an aerosol generation assembly comprising such an aerosol generation device and an external device.

### BACKGROUND OF THE INVENTION

Different types of aerosol generation devices are already known in the art. Generally, such aerosol generation devices comprise a storage portion for storing a vaporizable material, which can comprise for example a liquid or a solid. A heating system is formed of one or more electrically activated resistive heating elements arranged to heat said vaporizable material to generate the aerosol. The aerosol is released into a flow path extending between an inlet and an outlet of the aerosol generation device. The outlet may be arranged as a mouthpiece, through which a user inhales for delivery of the aerosol.

Generally, aerosol generation devices are designed so as to be easy to carry for a user. For example, they are relatively small in order to be stored in the user's pocket or bag when not in use. Typically, a user carries the aerosol generation device most of the time throughout the day to be able to use it at any time

Such aerosol generation devices may also be relatively expensive. Thus, they are valuable items that can attract thieves.

The aerosol generation devices known in the art are thus likely to arouse the envy of dishonest persons. There exist a significant risk that these devices might be stolen.

US 2015/181945 A1 discloses an electronic vaping device comprising a communication interface for communicating with a communication device external to the electronic vaping device over a communication link.

### SUMMARY OF THE INVENTION

One of the aims of the invention is to provide an aerosol generation device that provides better security for the device and for its user.

For this purpose, the invention relates to an aerosol generation device according to claim 1.

Thanks to these features, the aerosol generation device makes it possible to share its location to an external server when there is a risk that the device has been stolen. These features improve the security provided by the device. The location sharing enables the user to locate the aerosol generation device for example when it has been stolen or lost. Furthermore, these features make it possible to save energy. Indeed, the geolocation data are transmitted only when the first proximity sensing signal is no longer received by the aerosol generation device. This makes it possible to avoid transmitting geolocation data when it is not needed.

According to some embodiments, the aerosol generation device is according to claim 2.

Thanks to these features, the location sharing is carried out through a communication network that is easily available.

According to some embodiments, the aerosol generation device is according to claim 3.

According to some embodiments, the aerosol generation device is according to claim 4.

The millimeter-wave radar is used to detect moving objects around the user. This can, for example, be of use when the user walks through a dangerous area or an area with low visibility. The aerosol generation device offers a way to detect threats such as, for example, a possibly threatening person moving towards the user. In addition, the radar can be deactivated when the user feels no need of using it. For example, this makes it possible to conserve battery life.

According to some embodiments, the aerosol generation device is according to claim 5.

Thanks to these features, the user can point a direction to be monitored by the radar.

According to some embodiments, the aerosol generation device is according to claim 6.

According to some embodiments, the aerosol generating device is according to claim 7.

Thanks to these features, the aerosol generation device can cover a wide range of area surrounding the user. Potential threats can be detected early and from various angle.

The invention also relates to an aerosol generation assembly according to claim 8.

Thanks to these features, the user can acknowledge the location of the aerosol generation device thanks to an external device belonging to him/her.

According to some embodiments, the aerosol generating assembly is according to claim 9.

According to some embodiments, the aerosol generating assembly is according to claim 10.

According to some embodiments, the aerosol generation assembly is according to claim 11.

According to some embodiments, the aerosol generating assembly is according to claim 12.

Thanks to these features, the user can acknowledge that there is a risk that the aerosol generation device has been stolen. The warning signal makes it possible to make the user aware of this risk. Information on the aerosol generation device's location can be shared with the user through the external device.

According to some embodiments, the aerosol generation assembly is according to claim 13.

Thanks to these features, the security of the aerosol generation device is enhanced while the power consumption increase for the aerosol generation device and the external device is limited.

According to some embodiments, the aerosol generation assembly is according to claim 14.

Thanks to these features, the aerosol generation assembly comprises objects that are highly likely to be carried by the user most of the time.

The invention also relates to an aerosol generation device comprising:
- a heater configured to heat a vaporizable material to generate aerosol;
- a battery configured to power the heater; and
- at least a millimeter-wave radar configured to detect moving objects around the aerosol generation device and to generate detection data representative of a position of the moving objects.

The millimeter-wave radar is used to detect moving objects around the user. For example, this can be of use when the user walks through a dangerous area or an area with low visibility. The aerosol generation device offers a way to detect threats such as, for example, a possibly threatening person moving towards the user.

According to some embodiments, the millimeter-wave radar is configurable between a scanning configuration in which the millimeter-wave radar is able to detect moving objects and generate detection data representative of a position of the moving objects and an idle configuration in which it is disabled.

According to some embodiments, the aerosol generation device further comprises a switch system configured to command the millimeter-wave radar between the scanning configuration and the idle configuration, the switch system being controllable by a user of the device.

Thanks to these features, the radar can be deactivated when the user feels no need of using it. For example, this makes it possible to conserve battery life.

According to some embodiments, the aerosol generation device comprises a device body extending longitudinally along a device axis between two ends, the millimeter-wave radar being located at one of the two ends of the device body.

Thanks to these features, the user can point a direction to be monitored by the radar.

According to some embodiments, the millimeter-wave radar is a multi-angle radar designed to detect moving objects in several directions.

According to some embodiments, the millimeter-wave radar is designed to detect moving objects within a detection range from the radar, the detection range being comprised between 1 m and 10 m, preferably between 1 m and 20 m, advantageously between 1 m and 140 m.

Thanks to these features, the aerosol generation device can cover a wide range of area surrounding the user. Potential threats can be detected early and from various angle.

According to some embodiments, the aerosol generation device comprises:
- a geolocation chip configured to generate geolocation data representative of a position of the aerosol generation device; and
- a long-range antenna configured to transmit said geolocation data to an external server.

According to some embodiments, the long-range antenna is configured to transmit said geolocation data via a communication network.

Thanks to these features, the aerosol generation device makes it possible to share its location to an external server. These features further improve the security provided by the device. The location sharing enables the user to locate the aerosol generation device.

The invention also relates to an aerosol generation assembly comprising:
- an aerosol generation device as described above; and
- an external device,

the aerosol generation device further comprising a short-range antenna configured to transmit said detection data to the external device.

According to some embodiments, the external device further comprises a warning system configured to emit a warning signal when the external device receives said detection data.

According to some embodiments, the warning signal is a visual signal and/or an audible signal and/or a haptic signal, perceptible by a user.

Thanks to these features, the user can receive a warning from an external device. This warning makes the user aware of an incoming potential threat.

According to some embodiments, the external device further comprises a display system and a module for generating information on the display system, the module for generating information being configured to generate information representative of the detection data when the external device receives said detection data.

Thanks to these features, the external device pertaining to the user can display information on an incoming potential threat. For example, the user can know about the threat's position, speed, direction of movement, etc.

According to some embodiments, the external device is a connected object.

Thanks to these features, the aerosol generation assembly comprises objects that are highly likely to be carried by the user most of the time. Both the aerosol generation device and the connected object are used for a threat detection purpose.

### BRIEF DESCRIPTION OF THE DRAWINGS

- Figure 1 is a schematic diagram showing an aerosol generation assembly according to the invention and an external server configured to communicate with the aerosol generation assembly;
- Figure 2 is an illustration of an operation method performed by the aerosol generation assembly of figure 1, when the aerosol generation device has been stolen from the user; and
- Figure 3 is an illustration of an operation method performed by the aerosol generation assembly of figure 1, when a potential threat approaches the user.

### DETAILED DESCRIPTION OF THE INVENTION

Before describing the invention, it is to be understood that it is not limited to the details of construction set forth in the following description. It will be apparent to those skilled in the art having the benefit of the present disclosure that the invention is capable of other embodiments and of being practiced or being carried out in various ways.

As used herein, the term "aerosol **generation** device" or "device" may include a vaping device to deliver an aerosol to a user, including an aerosol for vaping, by means of aerosol generating unit (e.g. an aerosol generating element which generates vapor which condenses into an aerosol before delivery to an outlet of the device at, for example, a mouthpiece, for inhalation by a user). The device may be portable. "Portable" may refer to the device being for use when held by a user. The device may be adapted to generate a variable amount of aerosol, e.g. by activating a heater system for a variable amount of time (as opposed to a metered dose of aerosol), which can be controlled by a trigger. The trigger may be user activated, such as a vaping button and/or inhalation sensor. The inhalation sensor may be sensitive to the strength of inhalation as well as the duration of inhalation to enable a variable amount of vapor to be provided (so as to mimic the effect of smoking a conventional combustible smoking article such as a cigarette, cigar or pipe, etc.). The device may include a temperature regulation control to drive the temperature of the heater and/or the heated aerosol generating substance (aerosol pre-cursor) to a specified target temperature and thereafter to maintain the temperature at the target temperature that enables efficient generation of aerosol.

As used herein, the term "aerosol" may include a suspension of vaporizable material as one or more of: solid particles; liquid droplets; gas. Said suspension may be in a gas including air. Aerosol herein may generally refer to/include a vapor. Aerosol may include one or more components of the vaporizable material.

As used herein, the term **"vaporizable material"** or **"precursor"** or **"aerosol forming substance"** or **"substance"** is used to designate any material that is vaporizable in air to form aerosol. Vaporization is generally obtained by a temperature increase up to the boiling point of the vaporization material, such as at a temperature less than 400°C, preferably up to 350°C. The vaporizable material may, for example, comprise or consist of an aerosol-generating liquid, gel, wax, foam or the like, an aerosol-generating solid that may be in the form of a rod, which contains processed tobacco material, a crimped sheet or oriented strips of reconstituted tobacco (RTB), or any combination of these. The vaporizable material may comprise one or more of: nicotine, caffeine or other active components. The active component may be carried with a carrier, which may be a liquid. The carrier may include propylene glycol or glycerin. A flavoring may also be present. The flavoring may include Ethylvanillin (vanilla), menthol, Isoamyl acetate (banana oil) or similar.

As used herein, the term **"external device"** may refer to a device, which is able to establish a wireless data connection with the aerosol generation device as it is explained in the specification. Such an external device may be a mobile device like a mobile phone for example. Additionally, such an external device may be a smart device able to process at least some data received from the aerosol generation device or intended to be transmitted to the aerosol generation device. Such a smart device can be a smartphone, a smartwatch, a tablet computer, a laptop, a desktop computer or any other smart object implemented for example according to the loT ("Internet of things") technology.

### FIRST EMBODIMENT OF THE INVENTION

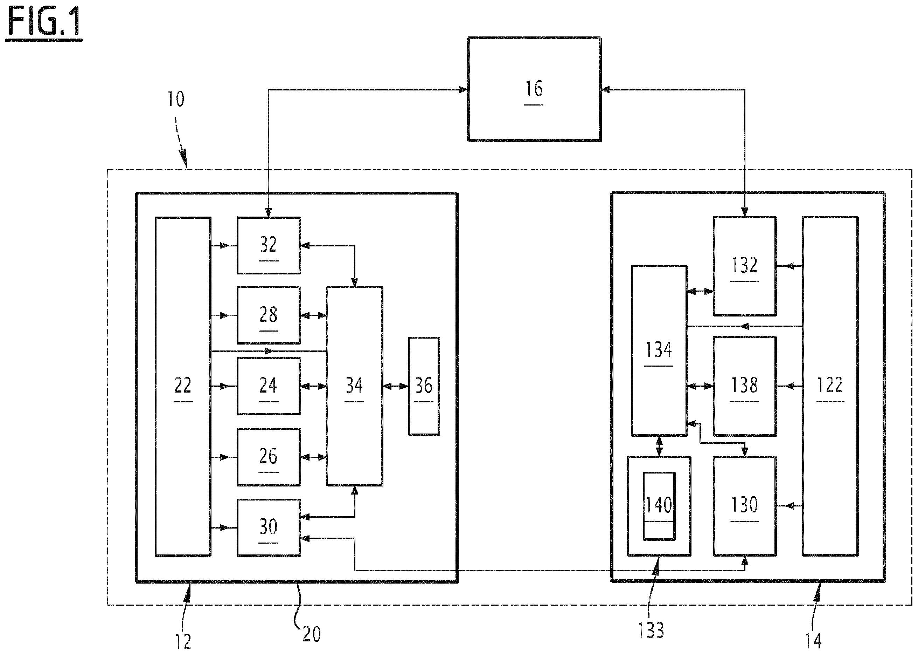

Referring to figure 1, an aerosol generation assembly 10 according to the first embodiment of the invention comprises an aerosol generation device 12 and an external device 14. The aerosol generation device 12 is configured to communicate with the external device 14. The aerosol generation device 12 and the external device 14 are further configured to communicate with an external server 16. For example, the external server 16 is a server accessible to law enforcement or to a private security service.

The aerosol generation device 12 comprises a body 20.

The device body 20 of the aerosol generation device 12 extends longitudinally along a device axis between two ends. The device body 20 delimits an interior part of the aerosol generation device 12 comprising a battery 22 configured to generate an electrical current, a heater 24 configured to heat a vaporizable material to generate aerosol, at least a millimeter-wave radar 26 configured to detect moving objects, a geolocation chip 28, a short-range antenna 30 configured to communicate with the external device 14, a long-range antenna 32 configured to communicate with the external server 16, a processing module 34 and a switch system 36.

The device body 20 of the aerosol generation device 12 can comprise other internal components performing different functionalities of the device known *per se.* It should also be noted that figure 1 presents only a schematic diagram of different components of the aerosol generation device 12 and does not necessarily show the real physical arrangement and dimensions of these components. Particularly, such an arrangement can be chosen according to the design of the aerosol generation device 12 and technical features of its components.

The battery 22 is for example a known battery designed to be charged using a power supply furnished by an external source. The battery 22 is further designed to provide a current of a predetermined intensity or voltage. The battery 22 is configured to power the heater 24, the radar 26, the geolocation chip 28, the short-range antenna 30, the long-range antenna 32, the processing module 34 and the switch system 36.

The heater 24 is in contact with a storage portion designed to store a vaporizable material used to generate aerosol. In a variant, the heater 24 is integrated partially into said storage portion. Based on the nature of the vaporizable material, the storage portion is designed to store the vaporizable material in a liquid and/or solid form. The storage portion is for example a pod or capsule containing e-liquid or a consumable such as a tobacco rod. The heater 24 is configured to heat the vaporizable material to an aerosol forming temperature in order to generate aerosol. The operation of the heater 24 is controlled by the processing module 34.

The millimeter-wave radar 26 is configured to detect moving objects around the aerosol generation device 12. The radar 26 is further configured to generate detection data representative of a position of the moving objects. For example, said detection data comprises spatial coordinates of the moving objects' location. The radar 26 is configurable between a scanning configuration, in which the radar 26 is able to detect moving objects and generate detection data and an idle configuration, in which the radar 26 is disabled. For example, when the radar 26 is in scanning configuration, the radar 26 is configured to automatically switch to idle configuration after a period of time. The period of time is for example comprised between 1 s and 60 s, advantageously between 5s and 30s. The radar 26 is connected to the processing module 34 and is configured to transmit said detection data to the processing module 34. For example, the radar 26 is located at one of the two ends of the device body 20. This enables the user to point the radar 26 towards an area to be monitored by the radar 26. For example, the radar 26 is a multi-angle radar designed to detect moving objects in several directions. This enables the covering of a wide area around the aerosol generation device 12 by the radar 26. The radar 26 is designed to detect moving objects within a detection range from the radar 26. For example, the detection range is comprised between 1 m and 10 m, preferably between 1 m and 20 m, advantageously between 1 m and 140 m.

The geolocation chip 28 is configured to generate geolocation data representative of a position of the aerosol generation device 12. The geolocation chip 28 is connected to the processing module 34 and is configured to transmit said geolocation data to the processing module 34. Advantageously, the geolocation chip 28 is configured to generate said geolocation data when the short-range antenna 30 stops receiving a first proximity sensing signal from the external device 14.

The short-range antenna 30 of the aerosol generation device 12 is configured to transmit said detection data to the external device 14. The short-range antenna 30 of the aerosol generation device 12 is further configured to receive the first proximity sensing signal from the external device 14 when the aerosol generation device 12 is within a proximity range PR around the external device 14. The short-range antenna 30 of the aerosol generation device 12 is further configured to emit a second proximity sensing signal within the proximity range PR around the aerosol generation device 12. The short-range antenna 30 is connected to the processing module 34. As long as the short-range antenna 30 receives the first proximity sensing signal, the short-range antenna 30 sends a continuous connection signal to the processing module 34. The connection signal is a signal representative of an effective connection between the aerosol generation device 12 and the external device 14. For example, the first proximity sensing signal and/or the second proximity sensing signal is a Bluetooth signal, preferably a Bluetooth Low Energy (BLE) signal. Advantageously, the reception of the first proximity sensing signal by the short-range antenna 30 accounts for low energy consumption. For example, the proximity range PR is a distance comprised between 1 m and 10 m, advantageously between 1 m and 5 m.

The long-range antenna 32 of the aerosol generation device 12 is configured to transmit said geolocation data to the external server 16. In particular, the long-range antenna 32 of the aerosol generation device 12 is configured to transmit said geolocation data when the short-range antenna 30 of the aerosol generation device 12 does not receive the first proximity sensing signal. Advantageously, the long-range antenna does not transmit said geolocation data when the short-range antenna 30 of the aerosol generation device 12 receives the first proximity sensing signal. For example, the long-range-antenna 32 of the aerosol generation device 12 is configured to transmit said geolocation data via a first communication network. For example, the first communication network is a cellular network, for example, a 2G network, a 3G network, a 4G network or a 5G network. The long-range antenna 32 is connected to the processing module 34 is configured to receive said geolocation data from the processing module 34 to transmit it to the external server 16.

The processing module 34 is configured to control the heater 24. In particular, the processing module 34 is configured to control the heater 24 so that the heater 24 heats the vaporizable material to the aerosol forming temperature. The processing module 34 is for example configured to activate the operation of the heater 24 upon reception of a triggering signal. The triggering signal can be emitted by an "ON/OFF" button further to its activation by the user or for example a pressure sensor further to detection of a user's puff.

The processing module 34 is further configured to control the radar 26 between the scanning configuration and the idle configuration. When the radar 26 is in scanning configuration, the processing module 34 receives said detection data from the radar 26. The processing module 34 is further configured to transmit said detection data to the short-range antenna 30.

The processing module 34 is further configured to receive said geolocation data from the geolocation chip 28. The processing module 34 is further configured to transmit said geolocation data to the long-range antenna 32. When the short-range antenna 30 of the aerosol generation device 12 does not receive the first proximity sensing signal, the processing module 34 does not receive the connection signal from the short-range antenna 30. In a particular embodiment, when the processing module 34 does not receive the connection signal, the processing module 34 transmits said geolocation data to the long-range antenna 32.

The processing module 34 is further configured to control the short-range antenna 30. In particular, the processing module 34 is configured to control the short-range antenna 30 so that the short-range antenna 30 emits the second proximity sensing signal.

The processing module 34 is further configured to control the long-range antenna 32. In particular, the processing module 34 is further configured to control the long-range antenna 32 so that the long-range antenna 32 transmits said geolocation data to the external server 16.

The processing module 34 is, for example, a processor.

The switch system 36 is configured to command the radar 26 between the scanning configuration and the idle configuration. The switch system 36 is controllable by a user of the aerosol generation device 12. The switch system 36 is connected to the processing module 34 and is configured to order the processing module 34 to control the radar 26 between the scanning configuration and the idle configuration. For example, the switch system 36 may comprise a mechanical switch such as a pushbutton switch, a pressure switch, a slide switch. According to a variant, the switch system 36 is integrated into a tactile human-machine interface of the aerosol generation device 12. In some embodiments, the switch system 36 comprises an on/off button that can also generate the triggering signal intended to the processing module 34 to activate/deactivate the operation of the heater 34.

The external device 14 comprises a battery 122, a short-range antenna 130 configured to communicate with the aerosol generation device 12, a long-range antenna 132 configured to communicate with the external server 16, a memory 133, a processing module 134, a display system 138 and a warning system. The external device 14 is a connected object. As mentioned above, the external device 14 is for example a smartphone, a smartwatch or a tablet.

The battery 122 of the external device is for example a known battery designed to be charged using a power supply furnished by an external source. The battery 122 is further designed to provide a current of a predetermined intensity or voltage. The battery 22 is configured to power the short-range antenna 130, the long-range antenna 132, the processing module 134 and the display system 138.

The short-range antenna 130 of the external device 14 is configured to receive said detection data from the aerosol generation device 12. The short-range antenna 130 of the external device 14 is further configured to receive the second proximity sensing signal from the aerosol generation device 12 when the external device 14 is within the proximity range PR around the aerosol generation device 12. The short-range antenna 130 of the external device 14 is further configured to emit the first proximity sensing signal within the proximity range PR around the external device 14. As said above, the first proximity sensing signal is, for example, a Bluetooth signal, preferably a BLE signal. As long as the short-range antenna 130 receives the second proximity sensing signal, the short-range antenna 130 sends a continuous connection signal to the processing module 134. The connection signal is a signal representative of an effective connection between the aerosol generation device 12 and the external device 14. The short-range antenna 130 is further configured to transmit said detection data to the processing module 134.

The long-range antenna 132 of the external device 14 is configured to receive said geolocation data from the external server 16. For example, the long-range-antenna 132 of the external device 14 is configured to receive said geolocation data via a second communication network. For example, said second communication network is a cellular network, for example, a 2G network, a 3G network, a 4G network or a 5G network or a Wi-Fi network. The long-range antenna 132 is connected to the processing module 134. The long-range antenna 132 is configured to transmit said geolocation data to the processing module 134.

The memory 133 comprises an information generating module 140. The information generating module 140 is a module for generating information on the display system 138. The information generating module 140 is configured to generate information representative of said geolocation data on the display system 138. As explained above, the long-range antenna 32 of the aerosol generation device 12 transmits said geolocation data to the external server 16 when the short-range antenna 30 of the aerosol generation device 12 does not receive the first proximity sensing signal. When the short-range antenna 30 of the aerosol generation device 12 does not receive the first proximity sensing signal, this means that the short-range antenna 130 of the external device 14 does not receive the second proximity sensing signal. Therefore, the module 140 generates information representative of said geolocation data when the short-range antenna 130 of the external device 14 does not receive the second proximity sensing signal. The information generating module 140 is further configured to generate information representative of said detection data on the display system 138. The memory 133 and the processing module 134 are connected. The memory 133 is configured to be accessed by the processing module 134. For example, the information generating module 140 is a program code for generating information designed to be executed by the processing module 134.

The display system 138 is configured to display information readable by the user of the external device 14. For example, the display system is a screen. The display system 138 and the battery 122 are electrically connected. The display system 138 is controlled by the processing module 134.

When the short-range antenna 130 of the external device 14 does not receive the second proximity sensing signal, the processing module 134 does not receive the connection signal from the short-range antenna 130. When the processing module 134 does not receive the connection signal, the processing module 134 accesses the memory 133. The processing module 134 executes the program code of the information generating module 140. Information representative of said geolocation data is then displayed on the display system. This information is designed to be readable by a user. For example, this information enable the user to locate the aerosol generation device 12. For example, the information comprise spatial coordinates of the location of the aerosol generation device 12 or instructions for reaching said location. For example, the processing module 134 is a processor.

The warning system is configured to emit a warning signal when the short-range antenna 130 of the external device 14 does not receive the second proximity sensing signal. The warning system comprises a warning generation module comprised in the memory 133, the display system 138 and/or a speaker and/or a vibrating element. For example, the warning generation module is a program code for generating the warning signal designed to be executed by the processing module 134. The speaker is connected to the processing module 134 and is configured to be controlled by the processing module 134. The vibrating element is connected to the processing module 134 and is configured to be controlled by the processing module 134. When the short-range antenna 130 does not receive the second proximity sensing signal, this means that the distance between the aerosol generation device 12 and external device 14 is greater than the proximity range PR. For example, this means that the aerosol generation device 12 may have been stolen. The warning signal is a signal perceptible by the user. For example, the warning signal informs the user that the aerosol generation device 12 may have been stolen. For example, the warning signal is a visual signal and/or an audible signal and/or a haptic signal. The visual signal is emitted by the display system 138. The audible signal is emitted by the speaker. The haptic signal is emitted by the vibrating element. When the short-range antenna 130 of the external device 14 does not receive the second proximity sensing signal, the processing module 134 executes the program code of the warning generation module. A warning signal is then generated. The processing module 134 controls the display system 138 and/or the speaker and/or the vibrating element to emit the warning signal.

The warning system is further configured to emit the warning signal when the short-range antenna 130 of the external device 14 receives said detection data. When the processing module 134 receives said detection data from the short-range antenna 130 of the external device 14, the processing module 134 accesses and executes the program code of the information generating module 140. Information representative of said detection data is then displayed on the display system. This information are designed to be readable by a user. For example, this information enable the user to locate moving objects around him. For example, the information comprises spatial coordinates of the location of the moving objects.

An operation method performed by the aerosol generation assembly 10 according to the first embodiment of the invention will now be explained.

Initially, it is considered that the aerosol generation device 12 and the external device 14 are carried by a user. The millimeter-wave radar 26 is in idle configuration.

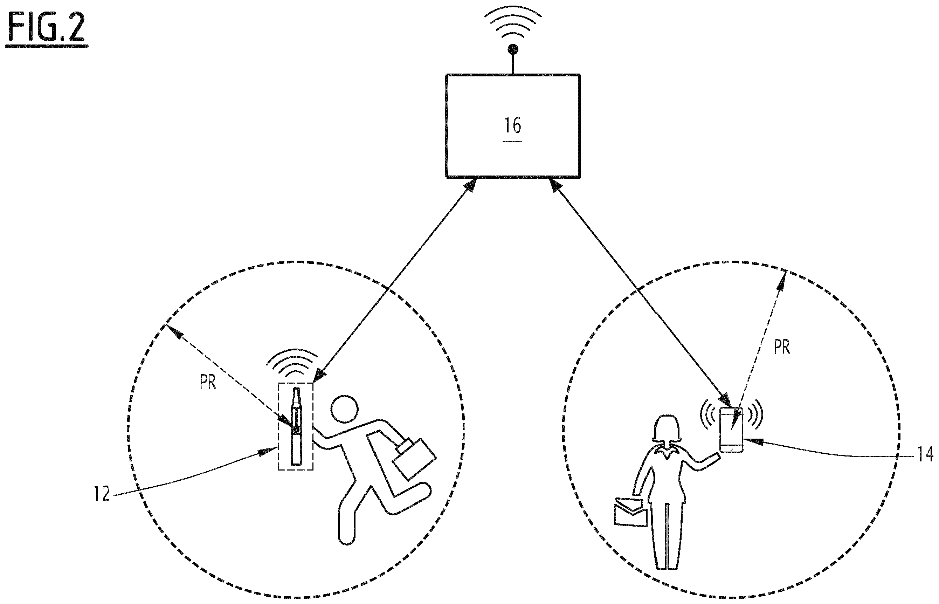

As illustrated on figure 2, when the distance between the aerosol generation device 12 and the external device 14 is greater than the proximity range PR, the short-range antenna 30 of the aerosol generation device 12 does not receive the first proximity sensing signal. Simultaneously, the short-range antenna 130 of the external device 14 does not receive the second proximity sensing signal. For example, this is the case when the aerosol generation device 12 has been stolen from the user. Advantageously, when the short-range antenna 30 of the aerosol generation device 12 does not receive the first proximity sensing signal, the geolocation chip 28 generates geolocation data representative of a position of the aerosol generation device 12. The long-range antenna 32 of the aerosol generation device 12 transmits said geolocation data generated by the geolocation chip 28 to the external server 16. Said geolocation data can be used by law enforcement to localize the aerosol generation device 12 in order to retrieve it. The long-range antenna 132 of the external device 14 receives said geolocation data from the external server 16 and the warning system of the external device 14 emits the warning signal. Information representative of said geolocation data is then displayed on the display system 138 of the external device 14. The user can then acknowledge the location of the potentially stolen aerosol generation device 12.

When the distance between the aerosol generation device 12 and the external device 14 is smaller than the proximity range PR, the short-range antenna 30 of the aerosol generation device 12 receives the first proximity sensing signal. Advantageously, as long as the short-range antenna 30 receives the first proximity sensing signal, the geolocation chip 28 does not generate said geolocation data. Thus, the proximity sensing signal is used permanently to assess a potential theft of the device 12 and the geolocation chip 28 is used only upon disruption of a proximity sensing link between the device 12 and the external device 14. This makes it possible to avoid using the geolocation chip 28, which can consumes a significant amount of energy, when it is not necessary, and only use it when the first proximity sensing signal is no longer received by the device 12.

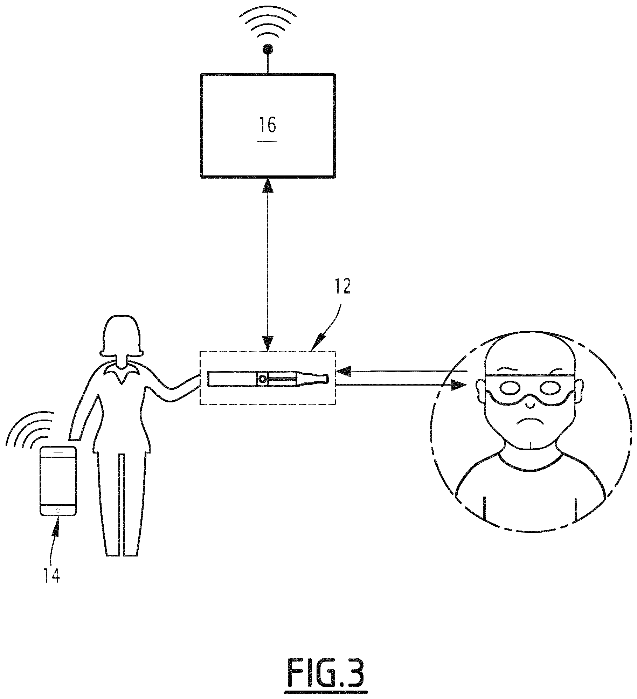

As illustrated on figure 3, when the user uses the aerosol generation device 12 in unsafe places, the user can control the switch system 36 of the aerosol generation device 12 to command the millimetre-wave radar 26 in scanning configuration.

The user points the end of the device body 20 where the radar 26 is located towards a potentially dangerous area to be monitored. The radar 26 detects moving objects around the aerosol generation device 12 and generates detection data representative of a position of the moving objects. The short-range antenna 30 of the aerosol generation device transmits said detection data to the external device 14. Information representative of said detection data are then displayed on the display system 138 of the external device 14. The user can then acknowledge the location of moving objects around him. In particular, the user can acknowledge the position of potential threats, like thieves, around him/her. This can prevent the aerosol generation device 12 from being stolen. Furthermore, this can provide better security for the user.

### SECOND EMBODIMENT OF THE INVENTION

An aerosol generation assembly 10 according to a second embodiment of the invention is described below. The second embodiment of the aerosol generation assembly 10 is similar to the first embodiment except the differences explained below.

According to the second embodiment of the invention, the long-range antenna 32 of the aerosol generation device 12 is further configured to transmit said detection data to the external server 16.

The external servers 16 then receives said detection data representative of potential threats around the user and said geolocation data representative of the location of the aerosol generation device 12. Said detection data can then be used by law enforcement to assess a potential threat situation for the user.

An operation method performed by the aerosol generation assembly 10 according to the second embodiment of the invention will now be explained. This operation method is similar to the operation method detailed above except the differences explained below.

As illustrated on figure 3, the long-range antenna 32 of the aerosol generation device 12 transmits detection data generated by the radar 26 to the external server 16.

## Claims

1. An aerosol generation device (12) comprising:
- a heater (24) configured to heat a vaporizable material to generate aerosol;
- a battery (22) configured to power the heater (24);
- a geolocation chip (28) configured to generate geolocation data representative of a position of the aerosol generation device (12); and
- a short-range antenna (30) configured to receive a first proximity sensing signal from an external device (14) when the aerosol generation device (12) is within a proximity range (PR) around the external device (14);
**characterized in that** the aerosol generation device (12) further comprises a long-range antenna (32) configured to transmit said geolocation data to an external server (16) when the short-range antenna (30) does not receive said first proximity sensing signal.

2. The aerosol generation device (12) according to claim 1, wherein the long-range antenna (32) is configured to transmit said geolocation data via a communication network.

3. The aerosol generation device (12) according to claim 1 or 2, further comprising at least a millimeter-wave radar (26) configured to detect moving objects around the aerosol generation device (12), the millimeter-wave radar (26) being configurable between a scanning configuration in which the millimeter-wave radar (26) is able to detect moving objects and an idle configuration in which it is disabled.

4. The aerosol generation device (12) according to claim 3, further comprising a switch system (36) configured to command the millimeter-wave radar (26) between the scanning configuration and the idle configuration, the switch system (36) being controllable by a user of the device (12).

5. The aerosol generation device (12) according to claim 3 or 4, comprising a device body (20) extending longitudinally along a device axis between two ends, the millimeter-wave radar (26) being located at one of the two ends of the device body (20).

6. The aerosol generation device (12) according to any one of claims 3 to 5, wherein the millimeter-wave radar (26) is a multi-angle radar designed to detect moving objects in several directions.

7. The aerosol generation device (12) according to any one of claims 3 to 6, wherein the millimeter-wave radar (26) is designed to detect moving objects within a detection range from the radar, the detection range being between 1 m and 10 m, preferably between 1 m and 20 m, advantageously between 1 m and 140 m.

8. An aerosol generation assembly (10), comprising:
- an aerosol generation device (12) according to any one of the preceding claims; and
- an external device (14) configured to receive said geolocation data from the external server (16).

9. The aerosol generation assembly (10) according to claim 8, wherein:
- the short-range antenna (30) of the aerosol generation device (12) is configured to emit a second proximity sensing signal within the proximity range (PR) around the aerosol generation device (12); and
- the external device (14) comprises a short-range antenna (130) configured to receive the second proximity sensing signal when the external device (14) is within the proximity range (PR) around the aerosol generation device (12).

10. The aerosol generation assembly (10) according to claim 9, wherein the external device (14) further comprises a warning system configured to emit a warning signal when the short-range antenna (130) of the external device (14) does not receive the second proximity sensing signal.

11. The aerosol generation assembly (10) according to claim 10, wherein the warning signal is a visual signal and/or an audible signal and/or a haptic signal, perceptible by a user.

12. The aerosol generation assembly (10) according to any one of claims 9 to 11, wherein the external device (14) further comprises a display system (138) and a module (140) for generating information on the display system (138), the module for generating information (140) being configured to generate information representative of said geolocation data when the short-range antenna (130) of the external device (14) does not receive the second proximity sensing signal.

13. The aerosol generation assembly (10) according to any one of claims 9 to 12, wherein the first proximity sensing signal and/or the second proximity sensing signal is a Bluetooth signal, preferably a Bluetooth Low Energy (BLE) signal.

14. The aerosol generation assembly (10) according to any one of claims 9 to 13, wherein the external device (14) is a connected object.

## Patentansprüche

1. Aerosolerzeugungsvorrichtung (12), umfassend:
- eine Heizung (24), die konfiguriert ist, um ein verdampfbares Material zu erhitzen, um Aerosol zu erzeugen;
- eine Batterie (22), die konfiguriert ist, um die Heizung (24) mit Strom zu versorgen;
- einen Geolokalisierungschip (28), der konfiguriert ist, um Geolokalisierungsdaten zu erzeugen, die repräsentativ für eine Position der Aerosolerzeugungsvorrichtung (12) sind; und
- eine Nahbereichsantenne (30), die konfiguriert ist, um ein erstes Näherungssensorsignal von einer externen Vorrichtung (14) zu empfangen, wenn die Aerosolerzeugungsvorrichtung (12) innerhalb eines Näherungsbereichs (PR) um die externe Vorrichtung (14) herum ist;
**dadurch gekennzeichnet, dass** die Aerosolerzeugungsvorrichtung (12) ferner eine Langstreckenantenne (32) umfasst, die konfiguriert ist, um die Geolokalisierungsdaten an einen externen Server (16) zu übertragen, wenn die Nahbereichsantenne (30) das erste Näherungssensorsignal nicht empfängt.

2. Aerosolerzeugungsvorrichtung (12) nach Anspruch 1, wobei die Langstreckenantenne (32) konfiguriert ist, um die Geolokalisierungsdaten über ein Kommunikationsnetzwerk zu übertragen.

3. Aerosolerzeugungsvorrichtung (12) nach Anspruch 1 oder 2, ferner umfassend mindestens ein Millimeterwellenradar (26), das konfiguriert ist, um sich bewegende Objekte um die Aerosolerzeugungsvorrichtung (12) herum zu erfassen, wobei das Millimeterwellenradar (26) zwischen einer Abtastkonfiguration, in der das Millimeterwellenradar (26) in der Lage ist, sich bewegende Objekte zu erfassen, und einer Leerlaufkonfiguration, in der es deaktiviert ist, konfigurierbar ist.

4. Aerosolerzeugungsvorrichtung (12) nach Anspruch 3, ferner umfassend ein Schaltsystem (36), das konfiguriert ist, um das Millimeterwellenradar (26) zwischen der Abtastkonfiguration und der Leerlaufkonfiguration zu steuern, wobei das Schaltsystem (36) durch einen Benutzer der Vorrichtung (12) steuerbar ist.

5. Aerosolerzeugungsvorrichtung (12) nach Anspruch 3 oder 4, umfassend einen Vorrichtungskörper (20), der sich längs einer Vorrichtungsachse zwischen zwei Enden erstreckt, wobei sich das Millimeterwellenradar (26) an einem der zwei Enden des Vorrichtungskörpers (20) befindet.

6. Aerosolerzeugungsvorrichtung (12) nach einem der Ansprüche 3 bis 5, wobei das Millimeterwellenradar (26) ein Mehrwinkelradar ist, das konstruiert ist, um sich bewegende Objekte in mehreren Richtungen zu erfassen.

7. Aerosolerzeugungsvorrichtung (12) nach einem der Ansprüche 3 bis 6, wobei das Millimeterwellenradar (26) konstruiert ist, um sich bewegende Objekte innerhalb eines Erfassungsbereichs von dem Radar zu erfassen, wobei der Erfassungsbereich zwischen 1 m und 10 m, vorzugsweise zwischen 1 m und 20 m, vorteilhafterweise zwischen 1 m und 140 m ist.

8. Aerosolerzeugungsanordnung (10), umfassend:
- eine Aerosolerzeugungsvorrichtung (12) nach einem der vorherigen Ansprüche; und
- eine externe Vorrichtung (14), die konfiguriert ist, um die Geolokalisierungsdaten von dem externen Server (16) zu empfangen.

9. Aerosolerzeugungsanordnung (10) nach Anspruch 8, wobei:
- die Nahbereichsantenne (30) der Aerosolerzeugungsvorrichtung (12) konfiguriert ist, um ein zweites Näherungssensorsignal innerhalb des Näherungsbereichs (PR) um die Aerosolerzeugungsvorrichtung (12) herum zu emittieren; und
- die externe Vorrichtung (14) eine Nahbereichsantenne (130) umfasst, die konfiguriert ist, um das zweite Näherungssensorsignal zu empfangen, wenn die externe Vorrichtung (14) innerhalb des Näherungsbereichs (PR) um die Aerosolerzeugungsvorrichtung (12) herum ist.

10. Aerosolerzeugungsanordnung (10) nach Anspruch 9, wobei die externe Vorrichtung (14) ferner ein Warnsystem umfasst, das konfiguriert ist, um ein Warnsignal zu emittieren, wenn die Nahbereichsantenne (130) der externen Vorrichtung (14) das zweite Näherungssensorsignal nicht empfängt.

11. Aerosolerzeugungsanordnung (10) nach Anspruch 10, wobei das Warnsignal ein visuelles Signal und/oder ein akustisches Signal und/oder ein haptisches Signal ist, das von einem Benutzer wahrnehmbar ist.

12. Aerosolerzeugungsanordnung (10) nach einem der Ansprüche 9 bis 11, wobei die externe Vorrichtung (14) ferner ein Anzeigesystem (138) und ein Modul (140) zum Erzeugen von Informationen auf dem Anzeigesystem (138) umfasst, wobei das Modul zum Erzeugen von Informationen (140) konfiguriert ist, um Informationen zu erzeugen, die repräsentativ für die Geolokalisierungsdaten sind, wenn die Nahbereichsantenne (130) der externen Vorrichtung (14) das zweite Näherungssensorsignal nicht empfängt.

13. Aerosolerzeugungsanordnung (10) nach einem der Ansprüche 9 bis 12, wobei das erste Näherungssensorsignal und/oder das zweite Näherungssensorsignal ein Bluetooth-Signal, vorzugsweise ein Bluetooth-Signal mit niedriger Energie (BLE), ist.

14. Aerosolerzeugungsanordnung (10) nach einem der Ansprüche 9 bis 13, wobei die externe Vorrichtung (14) ein verbundenes Objekt ist.

## Revendications

1. Dispositif de génération d'aérosol (12) comprenant :
- un dispositif de chauffage (24) configuré pour chauffer un matériau vaporisable afin de générer un aérosol ;
- une batterie (22) configurée pour alimenter le dispositif de chauffage (24) ;
- une puce de géolocalisation (28) configurée pour générer des données de géolocalisation représentatives d'une position du dispositif de génération d'aérosol (12) ; et
- une antenne à courte portée (30) configurée pour recevoir un premier signal de détection de proximité d'un dispositif externe (14) lorsque le dispositif de génération d'aérosol (12) se trouve dans une plage de proximité (PR) autour du dispositif externe (14) ;
**caractérisé en ce que** le dispositif de génération d'aérosol (12) comprend en outre une antenne longue portée (32) configurée pour transmettre lesdites données de géolocalisation à un serveur externe (16) lorsque l'antenne courte portée (30) ne reçoit pas ledit premier signal de détection de proximité.

2. Dispositif de génération d'aérosol (12) selon la revendication 1, dans lequel l'antenne longue portée (32) est configurée pour transmettre lesdites données de géolocalisation via un réseau de communication.

3. Dispositif de génération d'aérosol (12) selon la revendication 1 ou 2, comprenant en outre au moins un radar à ondes millimétriques (26) configuré pour détecter des objets en mouvement autour du dispositif de génération d'aérosol (12), le radar à ondes millimétriques (26) étant configurable entre une configuration de balayage dans laquelle le radar à ondes millimétriques (26) est capable de détecter des objets en mouvement et une configuration de repos dans laquelle il est désactivé.

4. Dispositif de génération d'aérosol (12) selon la revendication 3, comprenant en outre un système de commutation (36) configuré pour commander le radar à ondes millimétriques (26) entre la configuration de balayage et la configuration de repos, le système de commutation (36) pouvant être commandé par un utilisateur du dispositif (12).

5. Dispositif de génération d'aérosol (12) selon la revendication 3 ou 4, comprenant un corps de dispositif (20) s'étendant longitudinalement le long d'un axe de dispositif entre deux extrémités, le radar à ondes millimétriques (26) étant situé à l'une des deux extrémités du corps de dispositif (20).

6. Dispositif de génération d'aérosol (12) selon l'une quelconque des revendications 3 à 5, dans lequel le radar à ondes millimétriques (26) est un radar à angles multiples conçu pour détecter des objets en mouvement dans plusieurs directions.

7. Dispositif de génération d'aérosol (12) selon l'une quelconque des revendications 3 à 6, dans lequel le radar à ondes millimétriques (26) est conçu pour détecter des objets en mouvement dans une plage de détection à partir du radar, la plage de détection étant comprise entre 1 m et 10 m, de préférence entre 1 m et 20 m, avantageusement entre 1 m et 140 m.

8. Ensemble de génération d'aérosol (10) comprenant :
- un dispositif de génération d'aérosol (12) selon l'une quelconque des revendications précédentes ; et
- un dispositif externe (14) configuré pour recevoir lesdites données de géolocalisation du serveur externe (16).

9. Ensemble de génération d'aérosol (10) selon la revendication 8, dans lequel :
- l'antenne courte portée (30) du dispositif de génération d'aérosol (12) est configurée pour émettre un second signal de détection de proximité à l'intérieur de la plage de proximité (PR) autour du dispositif de génération d'aérosol (12) ; et
- le dispositif externe (14) comprend une antenne à courte portée (130) configurée pour recevoir le second signal de détection de proximité lorsque le dispositif externe (14) se trouve dans la plage de proximité (PR) autour du dispositif de génération d'aérosol (12).

10. Ensemble de génération d'aérosol (10) selon la revendication 9, dans lequel le dispositif externe (14) comprend en outre un système d'avertissement configuré pour émettre un signal d'avertissement lorsque l'antenne à courte portée (130) du dispositif externe (14) ne reçoit pas le second signal de détection de proximité.

11. Ensemble de génération d'aérosol (10) selon la revendication 10, dans lequel le signal d'avertissement est un signal visuel et/ou un signal audible et/ou un signal haptique, perceptible par un utilisateur.

12. Ensemble de génération d'aérosol (10) selon l'une quelconque des revendications 9 à 11, dans lequel le dispositif externe (14) comprend en outre un système d'affichage (138) et un module (140) pour générer des informations sur le système d'affichage (138), le module pour générer des informations (140) étant configuré pour générer des informations représentatives desdites données de géolocalisation lorsque l'antenne à courte portée (130) du dispositif externe (14) ne reçoit pas le second signal de détection de proximité.

13. Ensemble de génération d'aérosol (10) selon l'une quelconque des revendications 9 à 12, dans lequel le premier signal de détection de proximité et/ou le second signal de détection de proximité est un signal Bluetooth, de préférence un signal Bluetooth Low Energy (BLE).

14. Ensemble de génération d'aérosol (10) selon l'une quelconque des revendications 9 à 13, dans lequel le dispositif externe (14) est un objet connecté.
